# EUROPEAN PATENT APPLICATION

(11) **EP 4 389 734 A1**
(43) Date of publication of application: **26.06.2024**
(21) Application number: 22214879.3
(22) Date of filing: 20.12.2022
(51) Int. Cl.: C07C 67/04, C07C 69/157

(54) **PROCESS FOR MAKING 1-PHENYLETHYL ACETATE**

(71) Applicant: Givaudan SA, 1214 Vernier (CH)
(72) Inventor: The designation of the inventor has not yet been filed
(74) Representative: Global Patents

(57) **Abstract**

It is provided a process of making the compound of formula (I) 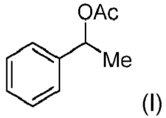 comprising the steps of
a) providing styrene,
b) converting styrene to the compound of formula (I) in the presence of a catalyst and acetic acid,
wherein the catalyst is selected from an alkali or an earth alkali metal salt or a sulfonic acid.

## Description

### TECHNICAL FIELD

The present invention relates generally to a process of making 1-phenylethyl acetate.

### BACKGROUND

1-phenylethyl acetate (CAS No. 93-92-5), also known as Gardenol, is a compound with a strong, radiant, green (leaf, apple) metallic (hazelnut, rhubarb) fruity (plumy, apricot) floral (gardenia) note. For example, the compound can be prepared in two steps by hydrogenation of acetophenone to 1-phenylethanol, followed by acetylation to 1-phenylethyl acetate.

Alternatively, Gardenol can be obtained from styrene (CAS No. 100-42-5). For example, this conversion can be performed with lanthanide salts as catalyst (Huaxue Shiji 2012, 34, 1151 - 1152), but the catalyst is rare and therefore expensive. Also a highly expensive and non-commercial tungsten-based catalysts (Synth.Comm. 2019, 49, 933-941) or cerium salts (J.Chem.Res. 2003, 5, 270-272) have been reported to be suitable for this process. In both cases a high catalyst load is required, and the amounts of acetic acid are not allowing for a safe and efficient process.

There is a need for new or improved processes for the preparation of Gardenol, for example in terms of efficiency and/or sustainability.

### SUMMARY

In accordance with a first aspect of the present invention there is provided a process of making the compound of formula (I)

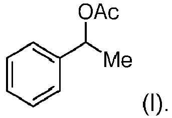

Certain embodiments of any aspect of the present invention may provide one or more of the following advantages:
- short synthesis - only one chemical step is required,
- broadly available, earth-abundant catalyst,
- low catalytic loading,
- no solvent required,
- fine-tuned amount of acetic acid leading to an overall safe process on industrial scale,
- acetic acid (starting material) available as a renewable feedstock,
- styrene (starting material) available as an upcycled starting material by conversion of plastic waste,
- recycling of the unreacted starting materials (acetic acid and styrene),
- high Gardenol olfactive quality,
- cost efficiency.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based on the surprising finding that the conversion of styrene to 1-phenylethyl acetate can be catalyzed by relatively cheap and widely available alkali or earth alkali metal salt or a sulfonic acid.

There is therefore provided herein a process of making 1-phenylethyl acetate (the compound of formula (I)), 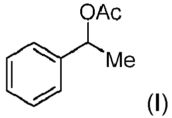 comprising the steps of
a) providing styrene,
b) converting styrene to the compound of formula (I) in the presence of a catalyst and acetic acid,
wherein the catalyst is selected from an alkali or an earth alkali metal salt or a sulfonic acid.

The method is described in Scheme 1

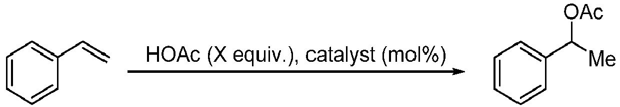

A catalyst selected from an alkali or an earth alkali metal salt or a sulfonic acid is relatively cheap and widely available. It is an improvement over prior art, in which rare, expensive, and sometimes also problematic catalysts (in terms of toxicity or handling safety) have been reported so far.

When the catalyst is an alkali or an earth alkali metal salt, it is for example an alkali or earth alkali halide, or the respective hydrate. For example, it can be selected from the group consisting of alkali or earth alkali chlorides, bromides or iodides, or the respective hydrates. In particular, it can be selected from the group consisting of LiCI, LiBr, Lil, NaBr, MgCl₂, MgBr₂, MgI₂, CaCl₂, CaBr₂, and CaI₂, or the respective hydrate, for example MgBr₂(H₂O)₆ or CaI₂(H₂O)ₓ.

When the catalyst is a sulfonic acid, it might be an aromatic sulfonic acid, for example a sulfonic acid selected from the group consisting of 2-naphthyl sulfonic acid (2-NSA) and *p-*toluenesulfonic acid (*p*TSA). Alternatively, the sulfonic acid can be a non-aromatic sulfonic acid, for example MeSO₃H or 10-camphorsulfonic acid (CSA). The sulfonic acid may also be a functional group immobilized on a resin, for example available as Amberlyst 36.

The present invention provides a viable synthesis, in particular for industrialization, while being highly sustainable, process and cost efficient. Compared to the current industrial process, the present invention offers an alternative by using styrene as starting material and transforming it in a single chemical step. The one-step synthesis of 1-phenylethyl acetate by addition of acetic acid across the double bond of styrene is already known in the prior art, and is using expensive, high molecular weight catalysts with additional handling issues. In contrast, the process according to the present invention does not require any solvent, uses a cheap and safe catalyst at low loading, and therefore generates a very limited amount of waste. The present process offers a very high atom-economy and generally satisfies the principles of green chemistry.

The use of a commercial, cheap and earth abundant alkali or an earth alkali metal salt catalyst at low loading to promote the reaction is advantageous. Such a catalyst allows for a good reactivity while limiting the loss of styrene, thereby reducing the atomic waste and the process costs. The balance between reactivity and limited styrene loss is essential to improve the process efficiency, as the formation of 1-phenylethyl acetate is highly reversible, but the degradation of styrene towards di-, oligo- and polymers is not.

In one example of the present invention, the catalyst is an alkali or an earth alkali bromide or the respective hydrate, for example LiBr, MgBr₂, CaBr₂, or MgBr₂(H₂O)₆.

The choice of the proper catalyst and adjustment of the further reaction parameters should take into account the availability, costs and loading of the catalyst, and potential styrene loss due to formation of polystyrene.

In a further embodiment of the process of the present invention, the catalyst is provided in an amount of 35 mol% or lower, 30 mol% or lower, 25 mol% or lower, 20 mol% or lower, 15 mol% or lower, 10 mol% or lower, 7.5 mol% or lower, 5.0 mol% or lower, preferably 2.5 mol% or lower, 1.0 mol% or lower, 0.75 mol% or lower, 0.5 mol% or lower.

For example, if the catalyst is a sulfonic acid, the amount of the catalyst is about 1.0 - 10.0 mol%, preferably 1.0 - 8.0 mol%, more preferably 1.0 - 3.0 mol% or 1.5 - 2.0 mol%.

For example, if the catalyst is an alkali or an earth alkali bromide, the amount of the catalyst is about 0.1 - 35 mol%, or 0.5 - 35 mol%, preferably 0.75 - 35 mol%, or 1.0 - 30.0 mol%, more preferably 2.5 - 20.0 mol% or 2.5 - 15.0 mol%, or 5.0 - 10.0 mol%, or about 7.5 mol%.

In a further embodiment of the process of the present invention, the amount of acetic acid is provided in at least 3 equivalents of styrene, preferably in at least 7 equivalents of styrene, or in at least 10 equivalents of styrene.

The formation of Gardenol from Styrene under the conditions of the present invention is reversible. It was found that a low excess of acetic acid allows to push the equilibrium of the reaction towards Gardenol, and therefore to limit the amount of Styrene in the reaction mixture and enhancing the yield of the desired product.

Furthermore, when applying the process of the present invention on industrial scale, it was found that styrene cannot be heated alone, as it shows a very high exothermal decomposition above 51 °C. It was found that dilution in acetic acid or in mixtures of acetic acid and Gardenol diminish the exothermic decomposition of styrene. With already 3 equivalents of acetic acid in the initial reaction mixture the reaction is safe to be performed at reflux, and mixtures of acetic acid and styrene can be recovered by distillation and then stored without safety concerns. Higher amounts of acetic acid will even more improve the safety of the reaction.

The amount of acetic acid needs to be balanced out by the costs of the unused material that needs to be washed out or recovered.

In a further embodiment of the process of the present invention, the catalyst is provided in an amount of 0.1 - 35 mol%, and the amount of acetic acid is provided in at least 3 equivalents of styrene.

In one example, the process of the present invention can be carried out with 20 mol% of CaCl₂ and with 20 equivalents of acetic acid.

In a further example, the process of the present invention can be carried out with 15 mol% of MgCl₂ and with 10 equivalents of acetic acid.

In a further example, the process of the present invention can be carried out with 0.75 mol% of MgBr₂(H2O)₆ and with 10 equivalents of acetic acid.

In a further example, the process of the present invention can be carried out with 1.5 mol% of 2-NSA and with 5 equivalents of acetic acid.

The conversion of styrene to Gardenol by the process of the present invention is carried out preferably at elevated temperature, for example at about 80 °C or higher, for example 100 °C or higher, or at about 110 °C or higher, or at about 120°C or higher. Temperatures above 120 °C might be applied, when the reaction is carried out under pressure, for example in an autoclave.

In general, also other temperature ranges can be used for the process of the present invention. Lower temperatures can slow down the formation of Gardenol, while not improving any other outcome of the reaction, therefore, they are not efficient. Higher temperatures require more energy and might increase styrene loss. The reaction time might be impacted by the other parameters, but is generally about 1 - 30 hours, preferably about 3-10 hours, for example 6 hours.

In general, also other reaction times might be considered for the process of the present invention. However, shorter reaction times might lead to incomplete conversion, while longer reaction times might lead to an increased loss of styrene.

The reaction of the present invention can be carried out under anhydrous conditions or in the presence of traces of water. For example, the hydrated form of a catalyst does not affect the reaction significantly. Water in higher quantities, for example about 1 equivalent, can already lower the yield of Gardenol and increases the styrene loss, although still providing the desired product.

In a further embodiment, the non-converted styrene and/ or non-converted acetic acid can be recycled from the reaction mixture by any suitable purification process, for example by distillation.

For example, styrene and acetic acid can be recovered as an azeotropic mixture by direct distillation of the reaction mixture. Said recovered mixture of styrene and acetic acid can be further used as starting material according to the process of the present invention. A shown in example 5, the use of the recovered mixture has no negative impact on the process of the present invention.

In a further embodiment of the present invention styrene is obtained as an upcycled starting material, for example as recycled styrene monomer from polystyrene waste. All atoms of styrene are incorporated in the final product, making thereby said part of Gardenol obtainable from upcycled material.

Alternatively, styrene can be obtained by dehydrogenation of ethylbenzene.

In a further embodiment of the present invention the acetic acid is obtained from renewable resources, for example from ethanol by oxidative fermentation, or by aerobic or anaerobic fermentation of sugars. All atoms of acetic acid are incorporated in the final product, making thereby said part of Gardenol obtainable from renewable resources.

Alternatively, acetic acid can be obtained by the carbonylation of methanol or other synthetic methods.

When the catalyst for the process of the present invention is a halide, for example an alkali halide or an earth alkali halide, (1-haloethyl)benzene can be formed as by-product in up to 2% yield from the addition of a halide atom to the double bond in place of the acetate in step b). These by-products render the purification by distillation more difficult in case their boiling point is close to the one of Gardenol, thereby contaminating a significant number of fractions. This is either lowering the olfactive quality of the product or the obtainable yield.

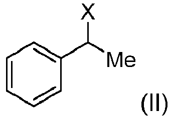

In a further embodiment of the process of the present invention, if the catalyst is a halide X, an acetate is added to the reaction mixture to remove the compound of formula (II). So the process of the present invention, wherein the catalyst is an alkali or earth alkali halide or the respective hydrate, is further comprising step c) which is eliminating byproduct (1-haloethyl)benzene formed during the method step b), by adding an acetate to the reaction mixture.

By the addition of the acetate, (1-haloethyl)benzene can be transformed in the same pot to 1-phenylethyl acetate in very short reaction time, for example, in about 30 min or less.

The so obtained reaction mixture allows to isolate Gardenol of highest level of olfactive purity, that is crucial for the use as fragrance ingredient. A final purification of Gardenol can be performed by distillation, if required.

For example, the acetate can be added to the initial reaction mixture. Alternatively, it can also be added at a later stage, for example after the formation of Gardenol.

The acetate can be selected from the group consisting of Zn(OAc)₂, NaOAc, CuOAc or the corresponding di-hydrates.

The acetate is added to the reaction mixture in an amount of at least 2 mol%, or of at least 2,5 mol% or of at least 5 mol%.

For example, when the catalyst for the process of the present invention is a bromide, for example an alkali bromide or an earth alkali bromide, (1-bromoethyl)benzene (compound of formula (Ila)) can be formed as by-product in up to 2% yield from the addition of a bromide atom to the double bond in place of the acetate in step b). This by-product renders the purification by distillation more difficult due to the boiling point being close to the one of Gardenol, thereby contaminating a significant number of fractions. This is either lowering the olfactive quality of the product or the obtainable yield.

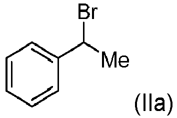

In a further embodiment of the process of the present invention, if the catalyst is a bromide, an acetate is added to the reaction mixture to remove the compound of formula (IIa). So the process of the present invention, wherein the catalyst is an alkali or earth alkali bromide or the respective hydrate, is further comprising step c) which is eliminating byproduct (1-bromoethyl)benzene formed during the method step b), by adding an acetate to the reaction mixture.

By the addition of the acetate, (1-bromoethyl)benzene can be transformed in the same pot to 1-phenylethyl acetate in very short reaction time, for example, in about 30 min or less.

The so obtained reaction mixture allows to isolate Gardenol of highest level of olfactive purity, that is crucial for the use as fragrance ingredient. A final purification of Gardenol can be performed by distillation, if required.

For example, the acetate can be added to the initial reaction mixture. Alternatively, it can also be added at a later stage, for example after the formation of Gardenol.

The acetate can be selected from the group consisting of Zn(OAc)₂, NaOAc, CuOAc or the corresponding di-hydrates.

The acetate is added to the reaction mixture in an amount of at least 2 mol%, or of at least 2,5 mol% or of at least 5 mol%.

### EXAMPLES

### General:

### Characterization data for 1-phenylethyl acetate:

1H NMR (CDCl₃, 500 MHz) δ 7.37-7.26 (m, 5H), 5.88 (q, *J*=6.6 Hz, 1H), 2.07 (s, 3H), 1.53 (d, *J*=6.6 Hz, 3H) ppm.

13C NMR (CDCl₃, 125 MHz) δ 170.3 (s), 141.6 (s), 128.5 (2d), 127.8 (d), 126.1 (2d), 72.3 (d), 22.2 (q), 21.3 (q) ppm.

MS (El): 164 (M+•,18), 122 (92), 107 (40), 105 (85), 104 (100), 103 (31), 79 (29), 78 (31), 77 (48), 51 (29), 43 (88).

The characterization data correspond to the reported values (Eur. J. Org. Chem. 2007, 13, 2073-2077).

### Example 1: Synthesis of 1-phenylethyl acetate with MgBr₂(H₂O)₆ & Zn(OAc)₂(H₂O)₂

A solution of MgBr₂(H₂O)₆ (2.1 g, 7.2 mmol, 0.0075 equiv.) in acetic acid (577 g, 9.60 mol, 10 equiv.) was heated to reflux. Styrene (100 g, 960 mmol, 1.0 equiv.) was then added dropwise over 20 min. The resulting reaction mixture was stirred for 7 hours at reflux and then cooled to 90 °C. At this temperature, Zn(OAc)₂(H₂O)₂ (5.3 g, 24 mmol, 0.025 equiv.) was added portion wise, and the mixture was left stirring for 30 min at 90 °C. Direct distillation of the reaction media allowed to recover 601.3 g of a mixture of unreacted acetic acid and styrene (containing 55.3 g of styrene and 546 g of acetic acid). Further distillation yielded 1-phenylethyl acetate (58.7 g, 37% yield, 357 mmol) as a colorless oil.

### Example 2: Synthesis of 1-phenylethyl acetate with MgCl₂

A solution of MgCl₂ (6.9 g, 72 mmol, 0.15 equiv.) in acetic acid (288 g, 4.80 mol, 10 equiv.) was heated to reflux. Styrene (50 g, 480 mmol, 1.0 equiv.) was then added dropwise over 20 min. The resulting reaction mixture was stirred for 8 hours at reflux. Direct distillation of the reaction media allowed to recover 276.4 g of a mixture of unreacted acetic acid and styrene (containing 28.6 g of styrene and 247.8 g of acetic acid). The residue was then diluted with MTBE and water, the phases separated and the organic layer was further washed with water (2x), 10wt% Na₂CO₃ and brine. The combined organic layers were dried over MgSO₄ and filtered. Solvent removal provided a yellow liquid which after distillation gave 1-phenylethyl acetate (23.34 g, 30% yield, 142 mmol) as a colorless oil.

### Example 3: Synthesis of 1-phenylethyl acetate with 2-naphthalenesulfonic acid (2-NSA)

To a stirred solution of styrene (3.8 g, 37 mmol, 1 equiv.) in acetic acid (11 g, 0.18 mol, 5.0 equiv.) was added 2-NSA (0.16 g, 0.55 mmol, 0.015 equiv.). The resulting reaction mixture was heated to 85 °C and left stirring for 6 hours. The reaction was quenched with water and extracted with MTBE. The organic layer was further washed with water (2x), 10wt% Na₂CO₃ and brine. The combined organic layers were dried over MgSO₄ and filtered. Solvent removal provided a yellow liquid which delivered 1-phenylethyl acetate after purification by flash chromatography (SiO₂, heptane:MTBE 90:10) as a colorless oil (1.85 g, 11.3 mmol, 31%).

### Example 4: Synthesis of 1-phenylethyl acetate with CaCl₂

A solution of CaCl₂ (2.70 g, 24.4 mmol, 0.2 equiv.) and styrene (12.7 g, 122 mmol, 1.0 equiv.) in acetic acid (146 g, 2.44 mol, 20 equiv.) was heated to 110 °C and stirred for 15 hours. Direct distillation of the reaction media allowed to recover 142.8 g of a mixture of unreacted acetic acid and styrene (containing 5.3 g of styrene and 137.5 g of acetic acid). The residue was then diluted with MTBE and water, the phases separated and the organic layer was further washed with water (2x), 10wt% Na₂CO₃ and brine. The combined organic layers were dried over MgSO₄ and filtered. Solvent removal provided a colorless liquid which after distillation gave 1-phenylethyl acetate (8.6 g, 43% yield, 53 mmol) as a colorless oil.

### Example 5: Recycling experiment - Synthesis of 1-phenylethyl acetate with CaCl₂ using recycled acetic acid and styrene recovered from Example 4

To 128.48 g of a mixture of recycled acetic acid and styrene (containing 4.73 g styrene and 123.75 g of acetic acid) was added CaCl₂ (2.70 g, 24.4 mmol, 0.2 equiv.), fresh styrene (8.0 g, 77 mmol, 0.63 equiv.) and acetic acid (23 g, 0.38 mol, 3.2 equiv.). The resulting solution was heated to 110 °C and left stirring for 15 hours. Direct distillation of the reaction media allowed to recover 135.6 g of a mixture of unreacted acetic acid and styrene (containing 12.7 g of styrene and 131.4 g of acetic acid). The residue was then diluted with MTBE and water, the phases separated and the organic layer was further washed with water (2x), 10wt% Na₂CO₃ and brine. The combined organic layers were dried over MgSO₄ and filtered. Solvent removal provided a colorless liquid which after distillation gave 1-phenylethyl acetate (9.02 g, 45% yield, 55 mmol) as a colorless oil.

### Example 6: Screening of reaction conditions:

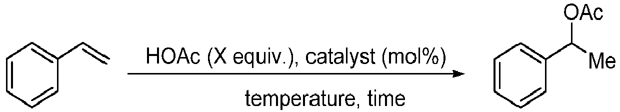

**Table 1**

| Entry | Catalyst (mol%) | HOAc [equiv.] | Temp [°C] | Time [h] | GC area % | |
|---|---|---|---|---|---|---|
| | | | | | Gardenol | Styrene |
| 1 | 2-NSA (2) | 10 | 80 | h | 34% | 55% |
| 2 | 2-NSA (1) | 5 | 80 | 6 | 37% | 54% |
| 3 | *p*TSA (8) | 20 | 80 | 6 | 49% | 22% |
| 4 | *p*TSA (1) | 12.5 | 100 | 3 | 34% | 56% |
| 5 | (20) | 10 | 120 | 5 | 35% | 61% |
| 6 | (20) | 20 | 110 | 15 | 48% | 47% |
| 7 | MgCl₂ (20) | 10 | 120 | 4 | 42% | 49% |
| 8 | MgCl₂ (20) | 6 | 120 | 5 | 36% | 48% |
| 9 | MgBr₂(H₂O)₆ (0.5) | 10 | 120 | 15 | 43% | 53% |
| 10 | MgBr₂(H₂O)₆ (0.75) | 10 | 120 | 7 | 39% | 57% |
| 11 | LiBr (20) | 20 | 110 | 15 | 53% | 40% |
| 12 | LiCI (20) | 20 | 110 | 15 | 35% | 61% |
| 13 | Lil (20) | 20 | 110 | 15 | 43% | 53% |
| 14 | NaBr (20) | 20 | 110 | 15 | 47% | 51% |
| 15 | CaBr₂(1) | 10 | 120 | 15 | 44% | 50% |
| 16 | CaI₂·(H₂O)ₓ (1) | 10 | 120 | 15 | 45% | 47% |
| 17 | FeCl₃ (20) | 20 | 110 | 15 | - | - |
| 18 | ZnCl₂ (20) | 20 | 110 | 15 | 7% | 5% |
| 19 | AlCl₃ (20) | 20 | 110 | 15 | 34% | 24% |
| 20 | MnCl₂ (5) | 10 | 120 | 15 | 11% | 84% |
| 21 | CoCl₂ (5) | 10 | 120 | 15 | 15% | 83% |
| 22 | CuCl₂ (5) | 10 | 120 | 15 | 1% | 96% |

Table 1 shows the results for the screening of different catalysts and reaction conditions, varying the catalyst and its amount, the amount of acetic acid, the reaction time and temperature. The reaction conditions are not optimized. It can be seen, that the reaction of the present invention can be carried out with a catalyst selected from an alkali or an earth alkali metal salt or a sulfonic acid to obtain a good yield of Gardenol whilst minimizing the loss of styrene. Other catalysts not belonging to the group of alkali or earth alkali metal salts or sulfonic acids proofed to be less efficient (entry 18, 20-22) and/or led to increased loss of styrene (entry 17-19).

## Claims

1. A process of making the compound of formula (I) 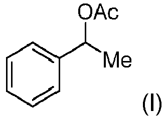 comprising the steps of
a) providing styrene,
b) converting styrene to the compound of formula (I) in the presence of a catalyst and acetic acid,
wherein the catalyst is selected from an alkali or an earth alkali metal salt or a sulfonic acid.

2. The process of claim 1, wherein the alkali or an earth alkali metal salt is an alkali or earth alkali halide or the respective hydrate.

3. The process of claims 1 or 2, wherein the alkali or an earth alkali metal salt is selected from the group consisting of LiCI, LiBr, Lil, NaBr, MgCl₂, MgBr₂, MgI₂, CaCl₂, CaBr₂, and CaI₂.

4. The process of claims 1 to 3, wherein the alkali or an earth alkali metal salt is an alkali or earth alkali bromide or the respective hydrate.

5. The process of claim 1, wherein the sulfonic acid is an aromatic sulfonic acid, preferably 2-naphthyl sulfonic acid or *p*-toluenesulfonic acid.

6. The process of any previous claim, wherein the catalyst is provided in an amount of 35 mol% or lower.

7. The process of any previous claim, wherein the acetic acid is provided in at least 3 equivalents of styrene.

8. The process of any previous claim, wherein the non-converted styrene is recycled from the reaction mixture by any suitable purification process.

9. The process of any previous claim, wherein the non-converted acetic acid is recycled from the reaction mixture by any suitable purification process.

10. The process of any previous claim, in which the styrene is obtained as an upcycled starting material.

11. The process of any previous claim, in which the acetic acid is obtained from renewable resources.

12. The process of claim 2, further comprising step c) which is eliminating byproduct (1-haloethyl)benzene formed during the method step b), by adding an acetate to the reaction mixture.

13. The process of claim 12, wherein the acetate is selected from the group consisting of Zn(OAc)₂, NaOAc, CuOAc or the corresponding di-hydrates.

14. The process of claim 12 or 13, wherein the acetate is added in an amount of at least 2 mol%.
